# EUROPEAN PATENT APPLICATION

(11) **EP 4 703 463 A1**
(43) Date of publication of application: **04.03.2026**
(21) Application number: 24797164.1
(22) Date of filing: 26.04.2024
(51) Int. Cl.: C12N 1/20

(54) **COMPOSITION FOR PROMOTING PROLIFERATION, DIFFERENTIATION AND MATURATION OF NEURAL STEM CELLS, PROMOTING MICROGLIAL ACTIVATION AND PROMOTING ASTROCYTE ACTIVATION, AND COMPOSITION FOR MAINTAINING AND/OR IMPROVING MEMORY AND/OR LEARNING ABILITY**

(30) Priority: 28.04.2023 JP 2023074562
(71) Applicant: Toa Biopharma Co., Ltd., Tokyo 151-0073 (JP); NATIONAL INSTITUTE OF ADVANCED INDUSTRIAL SCIENCE AND TECHNOLOGY, Tokyo 100-8921 (JP); The University of Tokyo, Bunkyo-ku, Tokyo 113-8654 (JP)
(72) Inventor: SATO Naoki, Tokyo 151-0073 (JP); NAMIHIRA Masakazu, Tsukuba-shi, Ibaraki 305-8560 (JP); HIRAYAMA Kazuhiro, Tokyo 113-8654 (JP); WATANABE Yohei, Tokyo 151-0073 (JP); HAYASHI Takuto, Tokyo 151-0073 (JP); INOUE Nana, Tokyo 151-0073 (JP); SAITO Ryuichi, Tokyo 151-0073 (JP); MASUDA Tomohide, Tokyo 151-0073 (JP)
(74) Representative: Krauss, Jan
(86) International application number: PCT/JP2024/016382
(87) International publication number: WO 2024/225419

(57) **Abstract**

A composition comprising one or more bacteria selected from lactic acid bacteria (e.g., Enterococcus faecium), butyrate-producing bacteria (e.g., Clostridium butyricum), and amylolytic bacteria (e.g., Bacillus subtilis) is disclosed. The composition is useful for various applications related to brain function, including: increasing the number of quiescent neural stem cells; promoting the proliferation, differentiation, and maturation of neural stem cells into neurons; promoting the maturation of immature neurons; promoting adult neurogenesis; modulating the activation state of microglia and astrocytes; maintaining homeostasis of brain function; and maintaining and/or improving memory and/or learning.

## Description

### Technical Field

The present disclosure relates to compositions for increasing the number of quiescent neural stem cells, promoting the differentiation and maturation of neural stem cells into neurons, promoting the proliferation of neural stem cells, promoting the maturation of immature neurons, promoting the activation of microglia, and promoting the activation of astrocytes, as well as compositions for maintaining and/or improving memory and/or learning ability.

This application claims priority to Japanese Application No. 2023-74562, which is incorporated herein by reference.

### Background Art

The function of the brain is established by neurons and glial cells (including microglia, astrocytes, and oligodendrocytes). Neurons are responsible for forming memory circuits, most of which are created during the embryonic period. Astrocytes structurally and functionally maintain the brain's neural circuits and the blood-brain barrier, while microglia maintain the homeostasis of the central nervous system as immune cells within the brain. Neural stem cells, which have the ability to differentiate into neurons and glial cells, are also present in the adult brain (particularly in the subventricular zone and the dentate gyrus (DG) of the hippocampus, and adult neurogenesis, the phenomenon in which neurons are generated from adult neural stem cells, has been reported (see Non-Patent Documents 1-3).

Although it has been reported that gut microbiota contributes to adult neurogenesis in the hippocampus, many details remain unclear. Adult neurogenesis is known to go through several stages from neural stem cells to mature neurons, but it has not been clarified at which stage intestinal bacteria act on neurogenesis or to what extent it contributes to the formation of new neurons. Furthermore, the mechanisms that induce neurogenesis remain poorly elucidated (see Non-Patent Documents 4-7).

Patent Document 1 discloses a "composition for maintaining and/or improving memory and learning ability containing cells of heterofermentative lactic acid bacilli of the genus Lactobacillus."

However, the composition of the present disclosure differs in components from the composition described in Patent Document 1.

### [Prior Art References]

### [Patent Documents]

[Patent Document 1] Patent JP6739603

### [Non-Patent Documents]

[Non-Patent Document 1] Cell Stem Cell. 2019 May 2;24(5):690-705.
[Non-Patent Document 2] Aging (Albany NY). 2021 Jul 29;13(14):18689-18700.
[Non-Patent Document 3] Nature. 2018 July; 559(7712):98-102.
[Non-Patent Document 4] Curr Opin Neurobiol. 2015 Feb;30:51-8.
[Non-Patent Document 5] Nat Med. 2019 Apr;25(4):554-560.
[Non-Patent Document 6] Front Neurosci. 2022 Oct 28;16:1030694.
[Non-Patent Document 7] Front Cell Dev Biol. 2020 May 29;8:407.
[Non-Patent Document 8] PLoS One. 2010 Jan 29;5(1):e8809.
[Non-Patent Document 9] Front Cell Neurosci. 2017 Aug 8;11:235.
[Non-Patent Document 10] Trends Mol Med. 2019 Nov;25(11):967-979.
[Non-Patent Document 11] Front Neurosci. 2022 Feb 16;16:824888.

### Summary of Invention

### Technical Problem

An object of the present disclosure is to elucidate how a composition containing lactic acid bacteria, butyrate-producing bacteria, and/or amylolytic bacteria, which improve the intestinal flora and the barrier function of the colon, affects brain function, and to provide a composition that induces and promotes adult neurogenesis, thereby contributing to the maintenance homeostasis of brain function and the maintenance and enhancement of memory and learning ability.

The inventors have conducted intensive research. They found that a composition containing lactic acid bacteria (particularly Enterococcus faecium), butyrate-producing bacteria (particularly Clostridium butyricum), and/or amylolytic bacteria(particularly Bacillus subtilis) has effects such as increasing the number of quiescent neural stem cells; promoting the proliferation of neural stem cells; promoting the differentiation and maturation of neural stem cells into neurons; promoting the maturation of immature neurons; promoting the activation of microglia; promoting the activation of astrocytes; promoting adult neurogenesis; and maintaining homeostasis of brain function, and thus completed the present disclosure. Furthermore, the inventors found that butyrate-producing bacteria have effects on maintaining and/or improving memory and/or learning, and thus completed the present disclosure.

The present disclosure is as follows:
1. A composition for one or more of the following uses, containing one or more selected from lactic acid bacteria, butyrate-producing bacteria, and amylolytic bacteria.
   1) For increasing the number of quiescent neural stem cells
   2) For promoting the proliferation of neural stem cells
   3) For promoting the differentiation and maturation of neural stem cells into neurons
   4) For promoting the maturation of immature neurons
   5) For promoting adult neurogenesis
   6) For maintaining and/or improving memory and/or learning ability
   7) For promoting the activation of microglia
   8) For promoting the activation of astrocytes
   9) For maintaining homeostasis of brain function
2. A composition according to item 1, containing two or more selected from lactic acid bacteria, butyrate-producing bacteria, and amylolytic bacteria.
3. A composition according to item 1, containing lactic acid bacteria, butyrate-producing bacteria, and amylolytic bacteria.
4. A composition according to any one of items 1 to 3, wherein the lactic acid bacteria are bacteria of the genus Enterococcus, the butyrate-producing bacteria are bacteria of the genus Clostridium, and the amylolytic bacteria are bacteria of the genus Bacillus.
5. A composition according to any one of items 1 to 3, wherein the lactic acid bacteria are Enterococcus faecium species or Enterococcus lactis species, the butyrate-producing bacteria are Clostridium butyricum species, and the amylolytic bacteria are Bacillus subtilis species.
6. A composition according to any one of items 1 to 3, wherein the lactic acid bacteria are Enterococcus faecium T-110, the butyrate-producing bacteria are Clostridium butyricum TO-A, and the amylolytic bacteria are Bacillus subtilis TO-A.
7. A composition for increasing the number of quiescent neural stem cells, containing Enterococcus faecium species or Enterococcus lactis species, Clostridium butyricum species, and Bacillus subtilis species.
8. A composition for promoting the differentiation and maturation of neural stem cells into neurons, containing Enterococcus faecium species or Enterococcus lactis species, Clostridium butyricum species, and Bacillus subtilis species.
9. A composition for promoting the proliferation of neural stem cells, containing Enterococcus faecium species or Enterococcus lactis species, Clostridium butyricum species, and Bacillus subtilis species.
10. A composition for promoting the maturation of immature neurons, containing Enterococcus faecium species or Enterococcus lactis species, Clostridium butyricum species, and Bacillus subtilis species.
11. A composition for promoting the activation of microglia, containing Enterococcus faecium species or Enterococcus lactis species, Clostridium butyricum species, and Bacillus subtilis species.
12. A composition for promoting the activation of astrocytes, containing Enterococcus faecium species or Enterococcus lactis species, Clostridium butyricum species, and Bacillus subtilis species.
13. A composition for promoting adult neurogenesis, containing Enterococcus faecium species or Enterococcus lactis species, Clostridium butyricum species, and Bacillus subtilis species.
14. A composition for maintaining homeostasis of brain function, containing Enterococcus faecium species or Enterococcus lactis species, Clostridium butyricum species, and Bacillus subtilis species.
15. A composition for maintaining and/or improving memory and/or learning ability, containing butyrate-producing bacteria.
16. A composition according to item 15, wherein the butyrate-producing bacteria are bacteria of the genus Clostridium.
17. A composition according to item 15, wherein the butyrate-producing bacteria are Clostridium butyricum TO-A.
18. A method of use for one or more of the following purposes, comprising administering a composition containing one or more selected from lactic acid bacteria, butyrate-producing bacteria, and amylolytic bacteria to a subject.
   1) For increasing the number of quiescent neural stem cells
   2) For promoting the proliferation of neural stem cells
   3) For promoting the differentiation and maturation of neural stem cells into neurons
   4) For promoting the maturation of immature neurons
   5) For promoting adult neurogenesis
   6) For maintaining and/or improving memory and/or learning ability
   7) For promoting the activation of microglia
   8) For promoting the activation of astrocytes
   9) For maintaining homeostasis of brain function
19. A method of use for maintaining and/or improving memory and/or learning ability, comprising administering a composition containing butyrate-producing bacteria to a subject.
20. Use of bacteria selected from lactic acid bacteria, butyrate-producing bacteria, and amylolytic bacteria in the production of one or more of the following compositions:
   1) For increasing the number of quiescent neural stem cells
   2) For promoting the proliferation of neural stem cells
   3) For promoting the differentiation and maturation of neural stem cells into neurons
   4) For promoting the maturation of immature neurons
   5) For promoting adult neurogenesis
   6) For maintaining and/or improving memory and/or learning ability
   7) For promoting microglial activation
   8) For promoting astrocyte activation
   9) For maintaining homeostasis of brain function
21. Use of butyrate-producing bacteria in the production of compositions for maintaining and/or improving memory and/or learning ability.
22. Food for the following uses, comprising the composition described in any of the preceding items 1 to 17:
   1) For increasing the number of quiescent neural stem cells
   2) For promoting the proliferation of neural stem cells
   3) For promoting the differentiation and maturation of neural stem cells into neurons
   4) For promoting the maturation of immature neurons
   5) For promoting adult neurogenesis
   6) For maintaining and/or improving memory and/or learning ability
   7) For promoting microglial activation
   8) For promoting astrocyte activation
   9) For maintaining homeostasis of brain function
23. The composition described in item 3, wherein the butyrate-producing bacteria are 100 to 2500 parts by mass and the amylolytic bacteria are 100 to 2500 parts by mass relative to 100 parts by mass of the lactic acid bacteria.

### Advantageous Effects of Invention

A composition of the present disclosure comprising at least lactic acid bacteria (particularly, Enterococcus faecium), butyrate-producing bacteria (particularly, Clostridium butyricum), and amylolytic bacteria (particularly, Bacillus subtilis), has one or more of the following effects:
1) Increase in the number of quiescent neural stem cells
2) Promotion of differentiation and maturation of neural stem cells into neurons
3) Promotion of neural stem cell proliferation
4) Promotion of maturation of immature neurons
5) Promotion of adult neurogenesis
6) Promotion of microglial activation
7) Promotion of astrocyte activation
8) Maintenance homeostasis of brain function

Additionally, the composition of the present disclosure, which includes at least butyrate-producing bacteria (particularly, Clostridium butyricum), has the effect of maintaining and/or improving memory and/or learning ability.

### Brief Description of Drawings

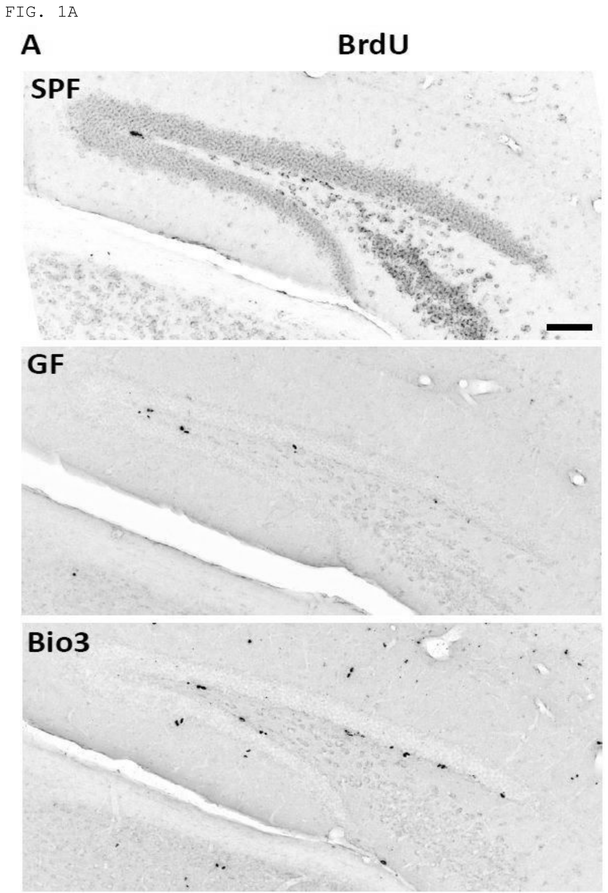

FIG. 1A shows immunostaining of BrdU-positive cells (black) in the hippocampal dentate gyrus region on the one day after the final day of BrdU administration in each group of mice. Scale bar = 100 µm.

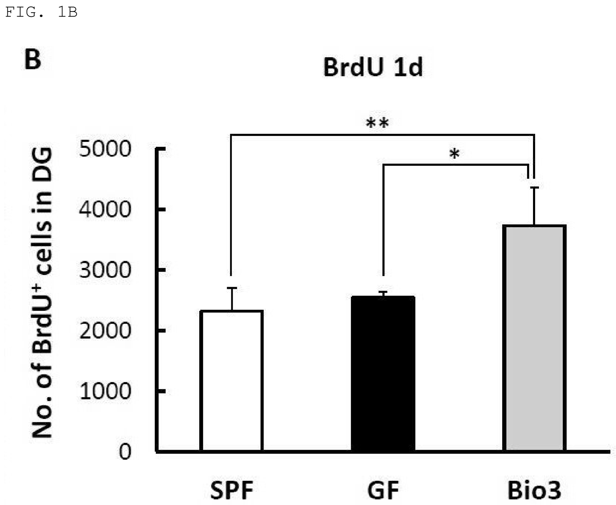

FIG. 1B is a graph showing the measured number of BrdU-positive cells in the granule cell layer of the dentate gyrus per individual mouse in each group. The group of mice administered Bio3 (Bio3) had statistically significantly more BrdU-positive cells compared to the SPF mice (SPF) and germ-free mice (GF) groups. *: p<0.05, **: p<0.01 (n=4 (SPF, Bio3), n=3 (GF)).

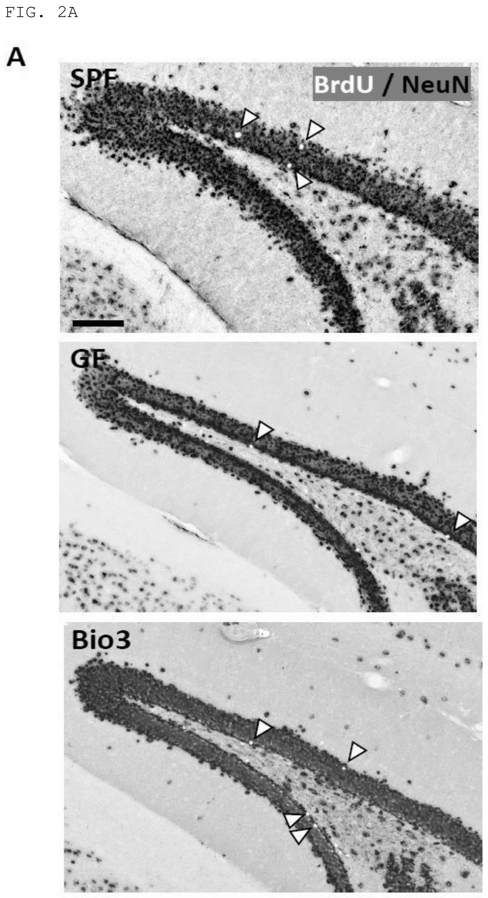

FIG. 2A shows immunostaining of BrdU-positive cells (white) and the expression of NeuN protein (black), which is specifically expressed in mature neurons, in the hippocampal dentate gyrus region on the 21st day after the final day of BrdU administration in each group of mice. Scale bar = 100 µm.

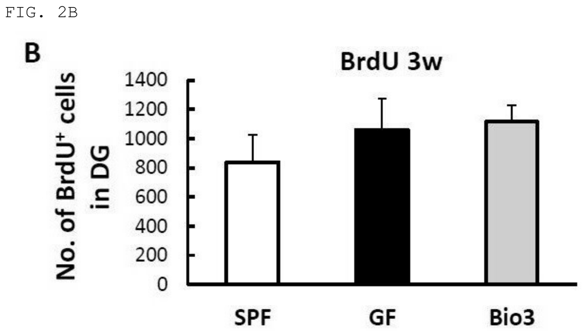

FIG. 2B is a graph showing the measured number of BrdU-positive cells in the granule cell layer of the dentate gyrus per individual mouse in each group. There was no statistically significant difference in the number of BrdU-positive cells between the groups. (n=4).

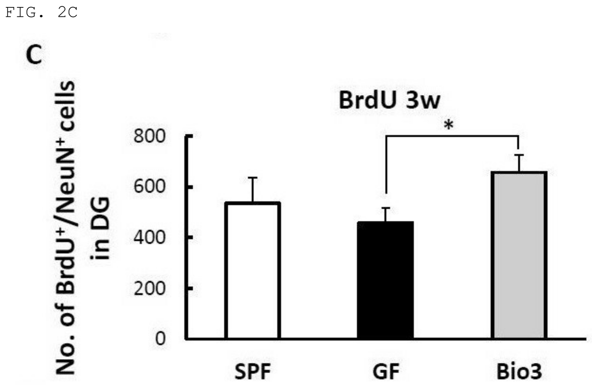

FIG. 2C is a graph showing the measured number of cells co-positive for BrdU and NeuN in the granule cell layer of the dentate gyrus per individual mouse in each group. The group of mice administered Bio3 (Bio3) had more cells co-positive for BrdU and NeuN compared to other groups, and the number was statistically significantly higher especially when compared to the GF group. *: p<0.05 (n=4).

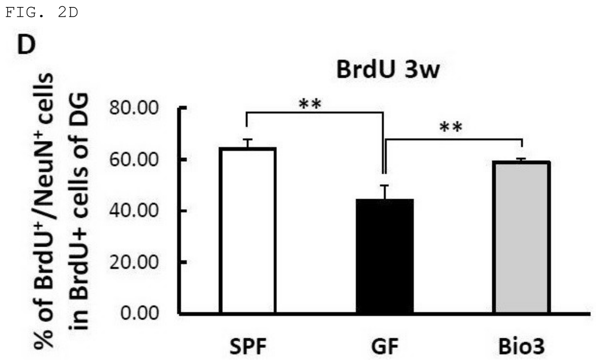

FIG. 2D shows the proportion of NeuN expression in BrdU-positive cells in each group. The group of mice administered Bio3 (Bio3) had a statistically significantly higher proportion of cells expressing NeuN compared to the GF mice group. **: p<0.01 (n=4).

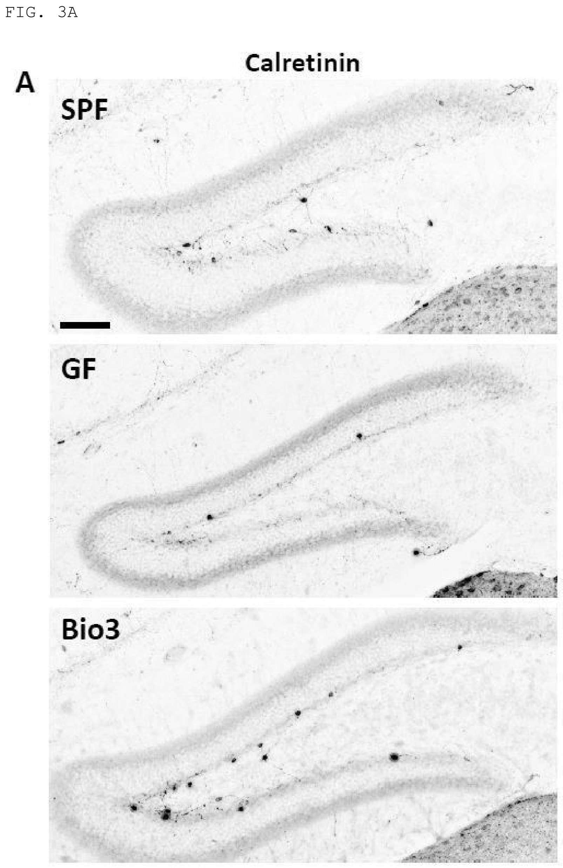

FIG. 3A shows immunostaining of the expression of Calretinin protein (black), which is specifically expressed in mature neurons, in the hippocampal dentate gyrus region on the 21st day after the final day of BrdU administration in each group of mice. Scale bar = 100 µm.

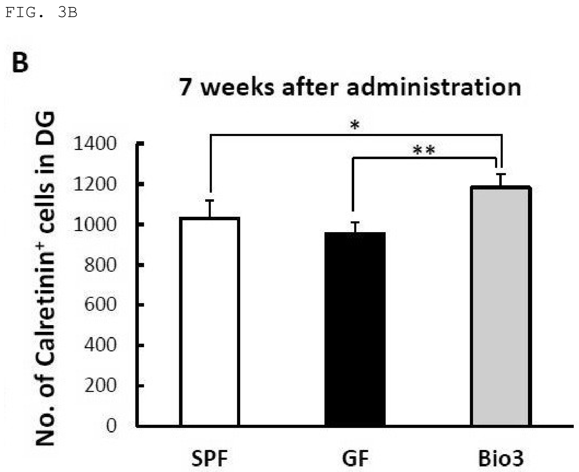

FIG. 3B is a graph showing the measured number of Calretinin-positive mature neurons in the granule cell layer of the dentate gyrus per individual mouse in each group. The Bio3 administration group had significantly more Calretinin-positive mature neurons compared to other groups. *: p<0.05, **: p<0.01 (n=4).

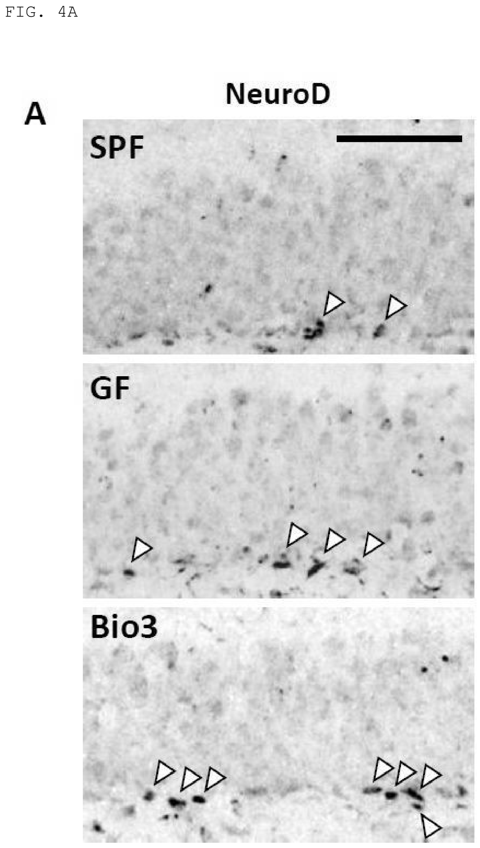

FIG. 4A shows immunostaining of the expression of the transcription factor NeuroD1 protein (NeuroD1, black, white arrowhead), which promotes the differentiation and maturation of neurons, in the hippocampal dentate gyrus region on the one day after the final day of BrdU administration in each group of mice. Scale bar = 50 µm.

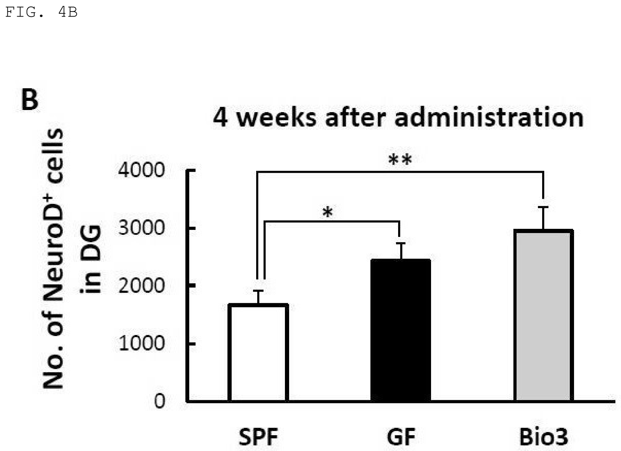

FIG. 4B is a graph showing the measured number of NeuroD1-positive cells in the granule cell layer of the dentate gyrus per individual mouse in each group. This graph shows the number of NeuroD1-positive cells measured after collecting and staining brain tissue from mice one day after the final day of BrdU administration (4 weeks after the start day of administration of Bio3 or saline). The number of NeuroD1-positive cells in the dentate gyrus was highest in the Bio3 administration group. *: p<0.05, **: p<0.01 (n=4).

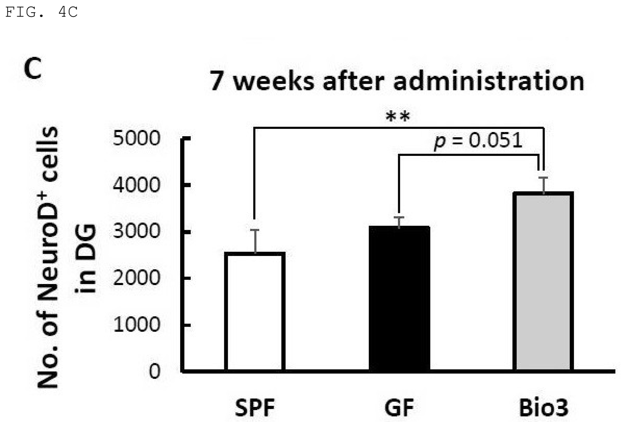

FIG. 4C is a graph showing the measured number of NeuroD1-positive cells in the granule cell layer of the dentate gyrus per individual mouse in each group. This graph shows the number of NeuroD1-positive cells measured after collecting and staining brain tissue from mice on the 21st days after the final day of BrdU administration (7 weeks after the start day of administration of Bio3 or saline). The number of NeuroD1-positive cells in the dentate gyrus was highest in the Bio3 administration group. *: p<0.05, **: p<0.01 (n=4).

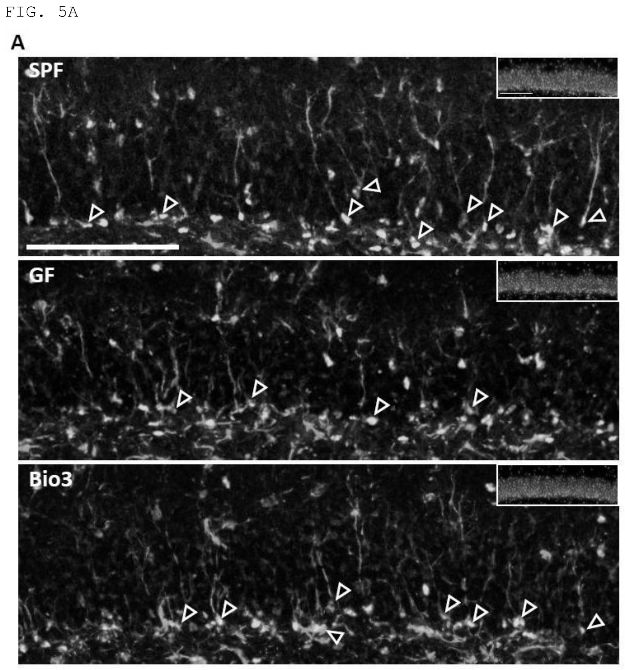

FIG. 5A shows quiescent neural stem cells (white-edged black arrowhead) co-expressing GFAP protein, nestin protein, and SOX2 protein in the hippocampal dentate gyrus region on the one day after the final day of BrdU administration (4 weeks after the start day of administration of Bio3 or saline). in each group of mice. Scale bar = 100 µm.

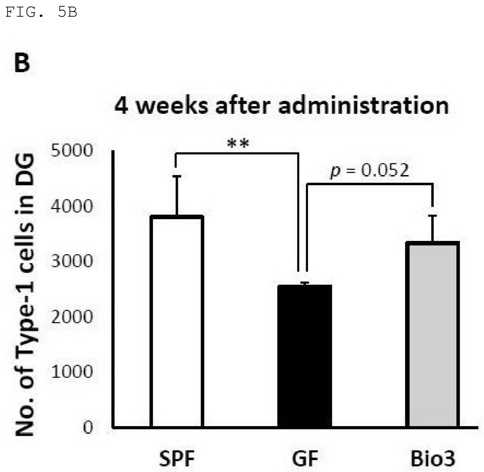

FIG. 5B is a graph showing the measured number of quiescent neural stem cells in the granule cell layer of the dentate gyrus per individual mouse in each group. The GF group had significantly fewer quiescent neural stem cells compared to the SPF group. However, in the Bio3 group, the number was similar to that of the SPF group. **: p<0.01 (n=4 (SPF), n=5 (GF, Bio3)).

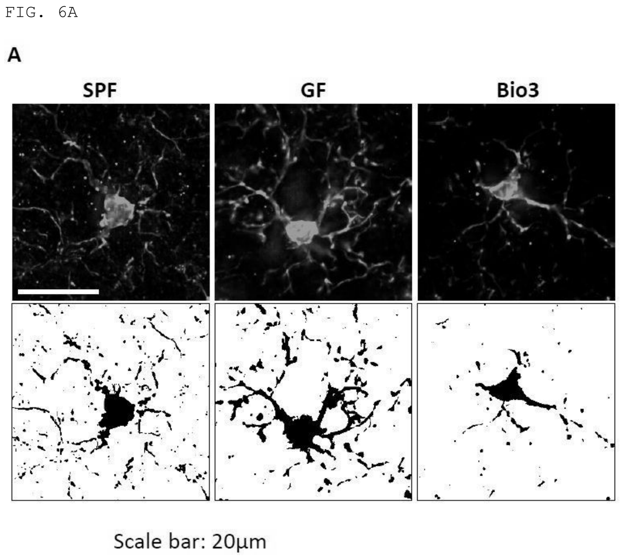

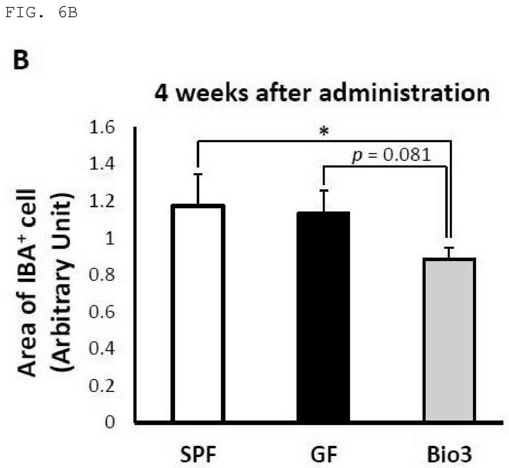

FIG. 6A shows images of microglia in the cerebral cortex of mice in each group on the one day after the final day of BrdU administration (4 weeks after the start day of administration of Bio3 or saline). The upper panel shows microglia expressing Iba1 protein (white). The lower panel is a fluorescence microscope image of Iba1-positive microglia that was binarized and converted to a black and white image, which was used for measuring cell area in FIG. 6B. Scale bar = 20 µm.
FIG. 6B is a graph showing the area of Iba1-positive microglia in the cerebral cortex of mice in each group, measured using ImageJ from the black and white images in FIG. 6A. The vertical axis values are arbitrary units (Arbitrary Unit) on ImageJ. The Bio3 group had a significantly reduced microglial area compared to the SPF group. *: p<0.05 (n=4 (SPF, Bio3), n=3 (GF)).

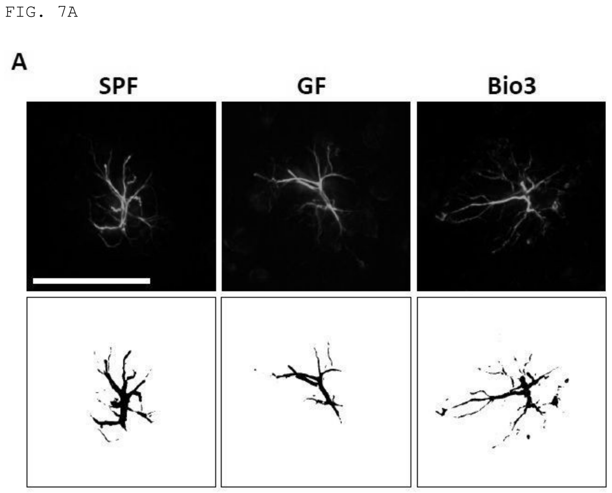

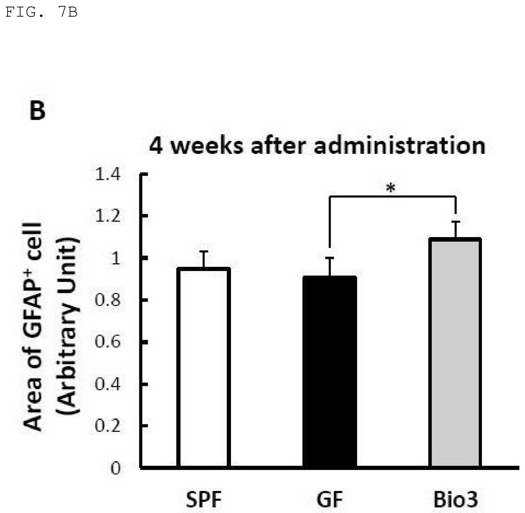

FIG. 7A shows astrocytes in the cerebral cortex of mice in each group on the one day after the final day of BrdU administration (4 weeks after the start day of administration of Bio3 or saline). The upper panel shows astrocytes expressing GFAP protein (white). The lower panel is a fluorescence microscope image of GFAP-positive astrocytes that was binarized and converted to a black and white image, which was used for measuring cell area in FIG. 7B. Scale bar = 50 µm.
FIG. 7B is a graph showing the average area of GFAP-positive astrocytes per individual in the cerebral cortex of mice in each group, measured using ImageJ from the black and white images in FIG. 7A. The vertical axis values are arbitrary units (Arbitrary Unit) on ImageJ. The Bio3 group had the largest astrocyte area compared to other groups, and it was statistically significantly larger especially compared to the GF group. *: p<0.05 (n=4).

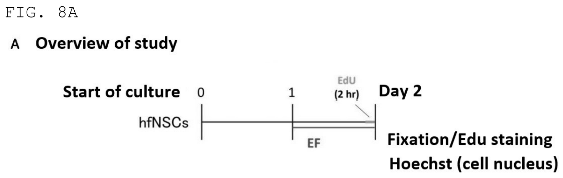

FIG. 8A shows an overview of the study.

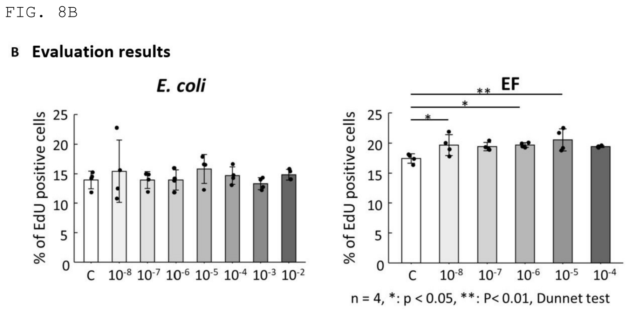

FIG. 8B shows the evaluation of proliferation.

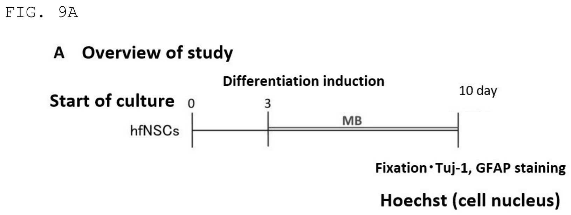

FIG. 9A shows an overview of the study.

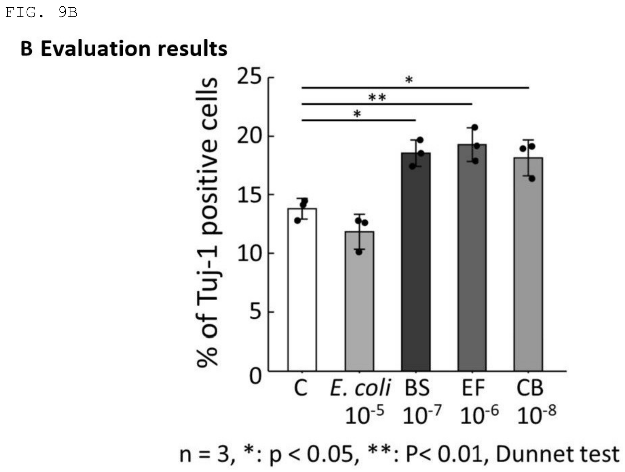

FIG. 9B shows the evaluation of differentiation into neurons.
FIG. 10A-1 shows an overview of the study - Evaluation agar medium.
FIG. 10A-2 shows an overview of the study - Flow from nematode starvation condition treatment to behavior observation.
FIG. 10B shows results - Chemotaxis index of nematodes to salt concentration.

### Description of Embodiments

The present disclosure relates to compositions for one or more of the following: increasing the number of quiescent neural stem cells; promoting the differentiation and maturation of neural stem cells into neurons; promoting the proliferation of neural stem cells; promoting the maturation of immature neurons; promoting microglial and astrocyte activation; promoting adult neurogenesis; maintaining homeostasis of brain function; and maintaining and/or improving memory and/or learning.

The present disclosure will be described in detail below. The compositions of the present disclosure are not particularly limited but refer to pharmaceutical compositions, food compositions, feed compositions, etc.

### (Composition of the Present Disclosure)

The composition of the present disclosure is a composition comprising one or more selected from lactic acid bacteria, butyrate-producing bacteria, and amylolytic bacteria.

### ○ Lactic Acid Bacteria

The lactic acid bacteria are preferably of the genus Enterococcus, more preferably of the species Enterococcus faecium or Enterococcus lactis, and even more preferably Enterococcus faecium T-110.

### ○ Butyrate-producing bacteria

The butyrate-producing bacteria are preferably of the genus Clostridium, more preferably of the species Clostridium butyricum, and even more preferably Clostridium butyricum TO-A.

### ○ Amylolytic bacteria

The amylolytic bacteria are preferably of the genus Bacillus, more preferably of the species Bacillus subtilis, and even more preferably Bacillus subtilis TO-A.

The above bacteria may be viable or inactivated (dead), but are preferably viable.

In addition, the compositions disclosed herein may be in the form of powders, granules, orally disintegrating tablets, plain tablets, coated tablets, capsules, or liquid formulations. The compositions of this disclosure are available from Toa Pharmaceutical Co., Ltd. (see: http://www.toabio.co.jp/medicalitem). Furthermore, Enterococcus faecium T-110, Clostridium butyricum TO-A, and Bacillus subtilis TO-A are available from the National Institute of Technology and Evaluation, Patent Microorganisms Depositary Center, under deposit numbers FERM BP-10867, FERM BP-10866, and FERM BP-07462, respectively.

In the literature "Int J Syst Evol Microbiol. 2021;71(8). DOI: 10.1099/ijsem.0.004948", it is reported that a specific clade of Enterococcus faecium (Clade B in the literature) belongs to the same cluster as the Enterococcus lactis strains based on phylogenetic analysis using core genome sequence comparison. The literature also discusses Enterococcus faecium T-110, indicating that it belongs to Clade B and significantly diverges from other Enterococcus faecium clades (Clade A1 and A2 in the literature), instead showing a very high similarity with the Enterococcus lactis strains, belonging to the same cluster (see Table 1 and Fig. 2 in the literature).

The content of lactic acid bacteria, butyrate-producing bacteria, and amylolytic bacteria in the compositions disclosed herein is not particularly limited, but examples can be illustrated as follows. For 100 parts by mass of lactic acid bacteria, the butyrate-producing bacteria can be 100 to 2500 parts by mass, 100 to 1500 parts by mass, 100 to 1000 parts by mass, 100 to 500 parts by mass, 100 to 300 parts by mass, or 100 to 200 parts by mass. For 100 parts by mass of lactic acid bacteria, the amylolytic bacteria can be 100 to 2500 parts by mass, 100 to 1500 parts by mass, 100 to 1000 parts by mass, 100 to 500 parts by mass, 100 to 300 parts by mass, or 100 to 200 parts by mass. The content of butyrate-producing bacteria and amylolytic bacteria can be selected and combined from the above ranges. For example, the butyrate-producing bacteria can be 100 to 2500 parts by mass, and the amylolytic bacteria can be 100 to 300 parts by mass.

### (Subjects for administration of the compositions disclosed herein)

The subjects for administration of the compositions disclosed herein are not particularly limited, but are organisms with neurons, preferably organisms with centralized nervous systems, more preferably vertebrates with a central nervous system, and even more preferably mammals (including humans). In the case of humans, both healthy individuals and patients are included. In particular, the intended subjects are organisms in which adult neurogenesis may occur; in humans, adults are targeted. However, given that compositions containing these strains have a history of safe use as oral pharmaceuticals for over 60 years from infants to the elderly, it is possible to target humans of all ages.

### (Methods of administration of the compositions disclosed herein)

The dosage, frequency, and interval of administration of the compositions disclosed herein are not particularly limited and may be appropriately selected according to conditions such as the purpose of prevention (particularly prevention of recurrence) and/or clinical treatment, disease type, patient's weight, age, and disease severity. For example, the following administration forms may be used, without limitation.
Powders (daily dose)
Lactic acid bacteria: 15-30 mg
Butyrate-producing bacteria: 75-150 mg
Amylolytic bacteria: 75-150 mg
Orally disintegrating tablets or uncoated tablets (daily dose) Lactic acid bacteria: 6-12 mg
Butyrate-producing bacteria: 30-60 mg
Amylolytic bacteria: 30-60 mg

It is preferable to mix these bacteria and administer them in the form of powders, granules, orally disintegrating tablets, uncoated tablets, coated tablets, capsules, or liquid formulations, and to administer them once or twice daily, or several times daily (e.g., morning, noon, and evening). Additionally, daily administration is preferred; however, administration once every few days, or one to several times every two weeks, is also possible. Furthermore, as a guideline for the daily intake of bacteria contained in the compositions disclosed herein-for example, in humans-the amount may be 10³ to 10²⁰ CFU/day, preferably 10⁵ to 10¹⁵ CFU/day, more preferably 10⁶ to 10¹³ CFU/day.

The compositions disclosed herein (or the use of the compositions) may serve as the following agents:
An agent for promoting adult neurogenesis, based on the action of increasing the number of quiescent neural stem cells, promoting the differentiation and maturation of neural stem cells into neurons, promoting the proliferation of neural stem cells, and/or promoting the maturation of immature neurons.

An agent for maintaining homeostasis of brain function, based on the action of promoting the activation of microglia and/or astrocytes.

An agent for maintaining and/or improving memory and/or learning ability based on the action of maintaining and/or improving memory and/or learning ability.

Furthermore, the compositions disclosed herein can be formulated into powders, tablets, granules, capsules, liquid formulations, etc., by blending with known carriers (e.g., starch, lactose, soy protein), excipients, binders, disintegrants, lubricants, stabilizers, suspending agents, and the like, using well-known methods.

The following examples are provided to describe the disclosure in detail, but the disclosure is not limited to these examples.

The following examples were conducted in accordance with the experimental animal handling guidelines of the University of Tokyo (Approval No. P20-130) and the National Institute of Advanced Industrial Science and Technology (Approval No. Animal 2022-0191).

### [Example 1]

### (Preparation of mice for each group)

In the following examples, three groups were prepared: specific-pathogen-free mouse group (SPF group), germ-free mouse group (GF group), and a group of GF mice colonized with a combination of three bacteria-lactic acid bacteria (Enterococcus faecium T-110: FERM BP-10867), butyrate-producing bacteria (Clostridium butyricum TO-A: FERM BP-10866), and amylolytic bacteria (Bacillus subtilis TO-A: FERM BP-07462)-referred to as the Bio3 group.

### (Breeding of GF group and Bio3 group)

### - Cage separation

Twelve-week-old GF mice (BALB/cA, purchased from CLEA Japan, Inc. and bred in-house) raised in germ-free conditions were divided into two groups and aseptically transferred from a vinyl isolator to a stainless-steel isolator for each group. One group received physiological saline by oral gavage as a control (GF group), and the other group was administered a mixture of the three bacteria described above-lactic acid bacteria, butyrate-producing bacteria, and amylolytic bacteria-by oral gavage (Bio3 group). The mice in each group were raised in the stainless-steel isolator until the day of dissection.

### - Administration of the three-bacteria mixture to the Bio3 group and saline administration to the GF group

The Bio3 group was orally administered a three-bacteria mixture in which each bacterial component (lactic acid bacteria, butyrate-producing bacteria, and amylolytic bacteria) was adjusted to 5×10⁸ CFU/mL, at 200 *µ*L per mouse, once per week for a total of five administrations. The GF group was similarly orally administered saline instead of the bacterial mixture.

### - Confirmation of the establishment of administered bacteria in the Bio3 group and confirmation of sterility in the GF group

Feces were collected on the 7th day after the 2nd and 4th oral administrations, respectively, and a homogenized suspension was immediately spread on agar plates of selective media for each of the lactic acid bacteria, butyrate-producing bacteria, and amylolytic bacteria and cultured. In parallel, smear slides were prepared from the suspension, and morphological identification was performed by Gram staining. The identification by Gram staining together with the detection of colonies on the respective selective media provided evidence that the administered bacteria were established in the intestines of the Bio3 group mice. Additionally, the GF group was also checked for GF status the day before dissection to confirm they were in an abacterial state.

### (Husbandry of SPF mouse group)

SPF mice (BALB/cA strain, Japan SLC) were maintained according to standard husbandry methods. The SPF group, GF group, and Bio3 group were matched for week-of-age for the intraperitoneal administration of bromodeoxyuridine (BrdU) and subsequent dissection described below.

### (Intraperitoneal administration of Bromodeoxyuridine (BrdU) to each mouse, followed by dissection and material collection)

### - Intraperitoneal administration of Bromodeoxyuridine (BrdU)

To monitor proliferating cells, BrdU was intraperitoneally administered to all mice in the study. Specifically, the mice in each group received BrdU at 50 mg/kg body weight, administered intraperitoneally once daily for three consecutive days, after 4 weeks (28 days) from the start of three-bacteria mixture or saline oral administration. BrdU (10 mg/mL), prepared by suspending in saline and sterilized with a 0.22 µm PES filter, was administered intraperitoneally at 150 µL using a 26.5-gauge needle with a 1 mL syringe. The whole brains of mice were collected at two time points, namely on the day after the final day of BrdU administration and three weeks thereafter, and processed for immunohistochemistry.

### - Acclimatization before dissection Before dissection

The mice were taken out of the stainless steel isolator with their cages, covered, and left in the laboratory for 3 hours before starting dissection.

### - Order of dissection

To avoid time differences between groups during dissection, each group was dissected alternately.

### - Dissection

Each mouse was subjected to general anesthesia with isoflurane, euthanized by exsanguination, and perfused with phosphate-buffered saline (PBS). Subsequently, perfusion fixation was performed using 4% paraformaldehyde (PFA), and the brain tissue was extracted. The extracted brain tissue was incubated in 4% PFA at 4°C overnight for further tissue fixation.

Subsequently, to prevent tissue damage during freezing, the 4% PFA containing brain tissue was replaced with a 15% sucrose solution and incubated overnight at 4°C, followed by replacement with a 30% sucrose solution and further incubation overnight at 4°C. The brain tissue was then embedded using optimal cutting temperature (OCT) compound (Sakura Finetek; 25608-930), frozen, and stored at - 80°C.

### (Tissue Immunostaining for the Brain)

Brain sections were prepared using a cryostat (Microm HM505e, Microm International GmbH, Walldorf, Germany) set to a thickness of 40 µm. The tissue sections were immersed in PBS solution to remove residual OCT compound and then stored at -25°C in a cryoprotectant solution (Bioenno Lifesciences; 006799-250) until staining.

Tissue immunostaining was performed as follows. After washing the tissue sections with PBS and 0.1% Triton X-100 solution diluted in PBS, a blocking solution (0.1% Triton X-100, 3% FBS in PBS) was added, and blocking was performed by incubation at room temperature for 1 hour. Next, the solution was replaced with a primary antibody solution diluted in the blocking solution, and antibody reaction was performed overnight at 4°C. After the primary antibody reaction, the sections were washed four times with 0.1% Triton X-100 solution, and a secondary antibody dilution solution containing Hoechst 33258 (0.5 µg/mL, Dojindo Laboratories; 343-07961) diluted in the blocking solution was used for antibody reaction for 3 hours at room temperature in the dark. After the secondary antibody reaction, the sections were washed four times with 0.1% Triton X-100 solution, dried, and mounted on Aminopropylsilane-coated slide glass (MATSUNAMI; S8443). After washing once with PBS, the sections were embedded with FluoroKeeper antifade reagent (Nacalai Tesque; 12593-64) and sealed with nail polish. The prepared slides were observed and photographed using a fluorescence microscope (THUNDER Imager 3D Cell Culture system; Leica Microsystems). For staining with anti-BrdU antibody, the tissue sections were washed with PBS, immersed in 2N HCl solution, treated at 37°C for 15 minutes, washed with PBS, immersed in the above blocking solution, and then tissue immunostaining was performed in the same manner as described above. For staining with anti-NeuroD1 antibody, after mounting the tissue sections on the above slide glass, they were washed with PBS, immersed in Target Retrieval (Dako; S1699), and antigen retrieval was performed by autoclaving (105°C for 15 minutes). After washing with PBS and 0.1% Triton X-100 solution, blocking was performed on the slide glass, followed by tissue immunostaining in the same manner as described above. The primary antibodies used for tissue immunostaining were rat anti-BrdU antibody (1:500, BIORAD; MCA6144), rabbit anti-NeuN antibody (1:500, Cell Signaling Technology; 24307S), chicken anti-DCX antibody (1:200, Abcam; ab153668), mouse Calretinin antibody (1:250, ProteinTech; 66496-1-IG), goat anti-NeuroD antibody (NeuroD, Santa Cruz; sc-1084), chicken anti-GFAP antibody (1:500, Abcam; ab4674), rat anti-NESTIN antibody (1:500, Wako; 012-26843), goat anti-SOX2 antibody (1:500, Abcam; ab239218), and rabbit anti-Iba1 antibody (1:500, Wako; 019-19741). The secondary antibodies used were CF568 anti-rat IgG antibody (1:500 dilution, Biotium; 20092), CF488 anti-mouse IgG antibody (1:500 dilution, Biotium; 20014), CF488 anti-rabbit IgG antibody (1:500 dilution, Biotium; 20015), CF568 anti-rabbit IgG antibody (1:500 dilution, Biotium; 20098), CF488 anti-chicken IgM antibody (1:500 dilution, Biotium; 20020), CF568 anti-goat IgG antibody (1:500 dilution, Biotium; 20106), and CF647 anti-goat IgG antibody (1:500 dilution, Biotium; 20048).

### Example 2

### (Effect of Promoting Neural Stem Cell Proliferation by Mixture of Three Bacteria)

The results of immunostaining for BrdU-positive cells in the dentate gyrus region of the hippocampus on the day after the final day of BrdU administration in each group of mice that received BrdU for three consecutive days are shown in Figures 1A and 1B.

The results in Figures 1A and 1B confirmed that the mixture of three bacteria (lactic acid bacteria, butyrate-producing bacteria, and amylolytic bacteria) promote the proliferation of neural stem cells, particularly those with proliferative capacity present in the dentate gyrus of the hippocampus.

### Example 3

### (Effect of Promoting Differentiation and Maturation of Neural Stem Cells into Neurons by Mixture of Three Bacteria)

The results of immunostaining for BrdU-positive cells and the expression of NeuN protein, which is specifically expressed in mature neurons, in the dentate gyrus region of the hippocampus on the 21st day after the final day of BrdU administration in each group of mice are shown in Figures 2A, 2B, 2C and 2D. The results in Figures 2A, 2B, 2C and 2D confirmed that the mixture of three bacteria of lactic acid bacteria, butyrate-producing bacteria, and amylolytic bacteria promote the differentiation and maturation of neural stem cells, particularly those present in the dentate gyrus of the hippocampus, into neurons.

### Example 4

### (Effect of Promoting Maturation of Immature Neurons by Mixture of Three Bacteria)

The results of immunostaining for Calretinin protein, which is specifically expressed in mature neurons, in the dentate gyrus region of the hippocampus on the 21st day after the final day of BrdU administration in each group of mice are shown in Figures 3A and 3B. The results in Figures 3A and 3B confirmed that the mixture of three bacteria of lactic acid bacteria, butyrate-producing bacteria, and amylolytic bacteria promote the maturation of immature neurons, particularly those present in the dentate gyrus of the hippocampus.

### Example 5

### (Effect of Promoting Differentiation and Maturation of Neural Stem Cells into Neurons via Increased Expression of NeuroD1 by Mixture of Three Bacteria)

The results of immunostaining for the expression of NeuroD1 protein, a transcription factor that promotes the differentiation and maturation of neurons, in the dentate gyrus region of the hippocampus in each group of mice are shown in Figures 4A, 4B and 4C. The results in Figures 4A, 4B and 4C confirmed that the mixture of three bacteria of lactic acid bacteria, butyrate-producing bacteria, and amylolytic bacteria promote the differentiation and maturation of neural stem cells, particularly those in the dentate gyrus, into neurons via increased expression of NeuroD1.

### Example 6

### (Effect of Recovering the Decrease in Quiescent Neural Stem Cell Number Due to germ-free condition by Mixture of Three Bacteria)

The results of immunostaining for quiescent neural stem cells co-expressing glial fibrillary acidic protein (GFAP) protein, neuroepithelial stem cell protein (Nestin), and sex-determining region Y-box 2 (SOX2) protein in the dentate gyrus region of the hippocampus in each group of mice are shown in Figures 5A and 5B. The results in Figures 5A and 5B confirmed that the mixture of three bacteria of lactic acid bacteria, butyrate-producing bacteria, and amylolytic bacteria recover the decrease in the number of quiescent neural stem cells due to germ-free condition (i.e., increase in the number of quiescent neural stem cells).

### Example 7

### (Effect of Promoting Microglial Activation by Mixture of Three Bacteria)

The results of immunostaining for microglia expressing ionized calcium-binding adapter molecule 1 (Iba1) protein in the cerebral cortex of mice in each group are shown in Figures 6A and 6B. It is known that microglia exhibit reduced cell body volume when activated. The results in Figures 6A and 6B confirmed that the mixture of three bacteria of lactic acid bacteria, butyrate-producing bacteria, and amylolytic bacteria promote microglial activation.

### Example 8

### (Effect of Promoting Astrocyte Activation by Mixture of Three Bacteria)

The results of immunostaining for GFAP-positive astrocytes in the cerebral cortex of mice in each group are shown in Figures 7A and 7B. It is known that astrocytes increase in cell volume when activated. The results in Figures 7A and 7B confirmed that the mixture of three bacteria of lactic acid bacteria, butyrate-producing bacteria, and amylolytic bacteria promote astrocyte activation.

### Example 9

### (Preparation of Human Neural Stem Cells and Culture Supernatants of Each Bacterium to Confirm the Effect of Each Single Bacterium)

The results shown in Examples 2 to 8 are the observations of brain cells in experiments where the three mixed bacteria of lactic acid bacteria, butyrate-producing bacteria, and amylolytic bacteria described in Example 1 were orally administered to mice. It was confirmed that each of these three bacteria alone also has an effect on neural stem cells and further shows effects on human cells using human neural stem cells. Human neural stem cells were prepared using a cell line derived from the human cerebral cortex of a 14-week-old male fetus, purchased from Phoenix Songs Biologicals, with informed consent obtained for tissue donation, and the preparation of test cells followed the method described in Japanese Patent No. 6976600 or international publication number WO 2019/009205. For the preparation of culture supernatants of each bacterium to be added to human neural stem cells, each bacterial strain was grown in a medium for human neural stem cells as described in patent number 6976600 or international publication number WO2019/009205. As for the culture method of each bacterial strain, Escherichia coli ATCC8739 (control strain) and amylolytic bacteria (Bacillus subtilis TO-A: FERM BP-07462, hereinafter BS) were inoculated into elbowed culture tubes and cultured under aerobic conditions at 37°C with linear shaking at 100-110 rpm, while lactic acid bacteria (Enterococcus faecium T-110: FERM BP-10867, hereinafter EF) and butyrate-producing bacteria (Clostridium butyricum TO-A: FERM BP-10866, hereinafter CB) were statically cultured under anaerobic conditions at 37°C.

The cultivation of each bacterial strain was terminated when the turbidity of the culture medium reached the stationary phase or just before, and the supernatant after centrifugation was sterilized using a 0.2 µm pore size filter. Tenfold serial dilutions of the supernatant were prepared, and each dilution was added to human neural stem cells, and the effects on the proliferation of neural stem cells shown in Example 10 and the effects on differentiation into neurons shown in Example 11 were evaluated respectively.

### Example 10

### (Effect of individual bacterial strains on the proliferation of human neural stem cells)

The presence or absence of effects on the proliferation of human neural stem cells by the culture supernatant of each bacterial strain was evaluated using the Click-iT EdU Cell Proliferation Kit (Thermo Fisher Scientific) following the recommended procedure of the kit. Specifically, human neural stem cells in an undifferentiated medium were cultured for one day with serial dilutions (10⁻² to 10⁻⁸) of each bacterial culture supernatant. Then 2.5 µM 5-ethynyl-2'-deoxyuridine (EdU) was added, and cells were cultured for 2 hours (see Fig. 8A). Subsequently, the cells were fixed with 4% PFA, and after the Click-iT reaction, the cell nuclei were stained with Hoechst 33342. The stained cells were visualized using a fluorescence microscope, and the proliferation (EdU labeling) index was calculated as the number of EdU-positive cells divided by the number of Hoechst-positive cells. As shown in the results of Fig. 8B, it was confirmed that the culture supernatant of EF significantly enhanced the proliferation of human neural stem cells. Specifically, the dilutions of EF at 10⁻⁵, 10⁻⁶, and 10⁻⁸ significantly enhanced the proliferation of human neural stem cells. In contrast, the culture supernatant of the control bacterial strain E. coli could not significantly enhance the proliferation of human neural stem cells.

### Example 11

### (Effect of individual bacterial strains on the differentiation of human neural stem cells into neurons)

Whether the culture supernatant of each bacterial strain induces differentiation of human neural stem cells into neurons was evaluated using indirect immunostaining for β-III tubulin protein, specifically expressed in differentiated neurons, i.e., Tuj-1. Specifically, human neural stem cells were first cultured for 3 days, after which serial dilutions (10⁻² to 10⁻⁸) of each bacterial culture supernatant were added and cells were further cultured for 7 days. The cells were then fixed with 4% PFA, and immunostained using an anti-Tuj-1 primary antibody (R&D Systems; MAB1195) and CF568 anti-mouse IgG secondary antibody (500-fold dilution, Biotium; 20105), along with Hoechst 33342 for nuclear staining (see Fig. 9A). The stained cells were visualized using a fluorescence microscope, and the neuronal differentiation index was calculated as the number of Tuj1-positive cells divided by the number of Hoechst-positive cells. As a secondary evaluation of the culture supernatant of selected bacterial strains, human neural stem cells cultured in a 24-well plate (0.6 x 10⁵ cells/well) were used, and all samples were evaluated simultaneously within a single plate. The results of the primary and secondary evaluations are shown in Figures 9A and 9B. As shown in the results of Fig. 9B, it was confirmed that the culture supernatants of BS, EF, and CB single strains, except for E. coli, significantly enhanced the induction of differentiation of human neural stem cells into neurons. Specifically, the dilutions of BS at 10⁻⁷, EF at 10⁻⁶, and CB at 10⁻⁸ significantly enhanced the induction of differentiation of human neural stem cells into neurons. In contrast, the culture supernatant of the control bacterial strain E. coli could not significantly enhance the induction of differentiation of human neural stem cells.

### Example 12

### (Effect of CB strain on memory and learning)

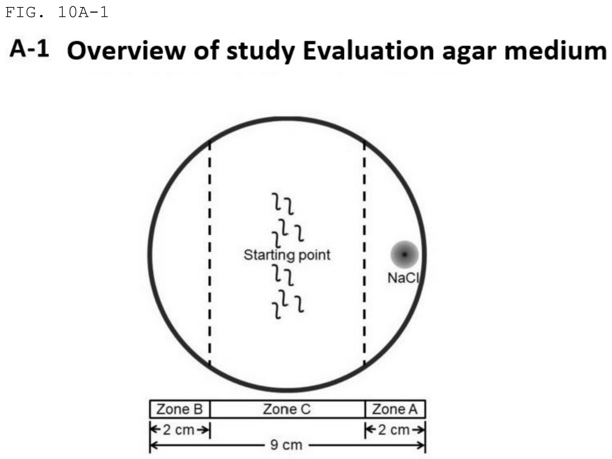

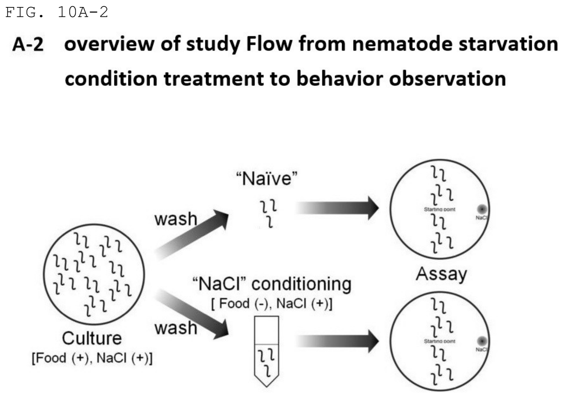

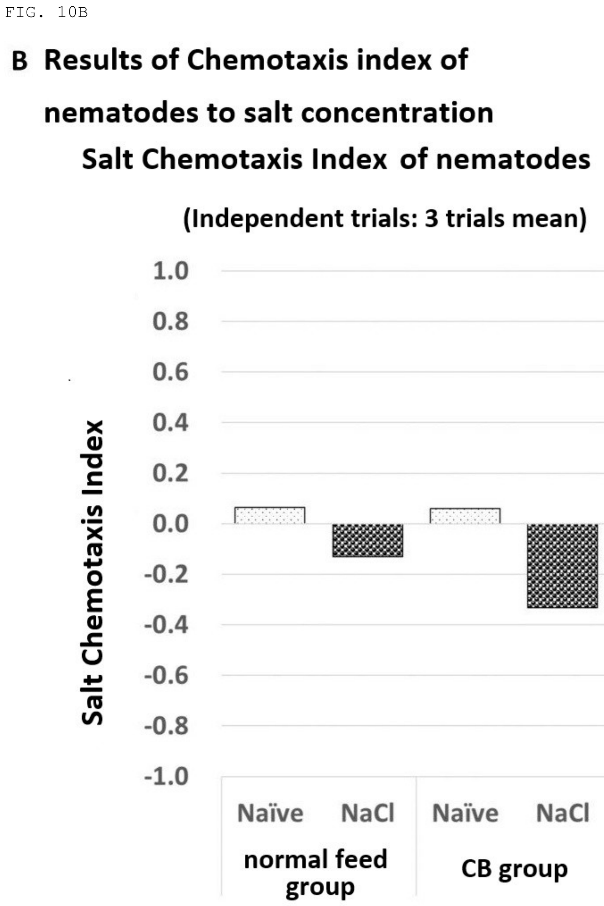

Caenorhabditis elegans is known to be able to memorize and learn the salt concentration in its environment. As an example, nematodes subjected to starvation memorize and learn the salt concentration in their breeding environment at that time and exhibit avoidance behavior towards the salt concentration environment experienced during starvation. Utilizing this characteristic, we established two groups from day 3 to day 7 of rearing (a four-day period): nematodes fed only the standard diet, Escherichia coli OP50 (normal diet group), and nematodes fed CB in addition to E. coli OP50 (CB test group). We then observed changes in starvation-associated avoidance behavior toward salt concentration. The test method and evaluation method were referenced from the literature "Neuron. 2006; 51: 613-25. DOI: 10.1016/j.neuron.2006.07.024." The specific evaluation was as follows. To generate a salt gradient on the evaluation agar plate (Fig. 10A-1), a cylindrical agar plug (5 mm in diameter) containing 100 mM NaCl was placed at one end of the plate and allowed to diffuse for 24 h. To enable nematodes to memorize and learn the presence or absence of salt in their breeding environment during starvation, two treatments were implemented (Fig. 10A-2): Naive (no starvation experience) and NaCl treatment (starvation in the presence of salt). Using the above evaluation agar plate and nematodes that underwent each treatment, behavior associated with memory and learning was observed. Specifically, nematodes were placed at the center of the plate and incubated for 1 h at 25° C; the numbers of nematodes present in the high-salt region (A), intermediate region (C), and low-salt region (B) were counted (Fig. 10A-1). The chemotaxis index (CI) toward salt concentration was calculated as (A - B) / (total - C), where CI approaching +1 indicates attraction to salt and CI approaching -1 indicates avoidance. Note that the NGM medium commonly used for normal nematode breeding, including this example, contains 50 mM NaCl, so it is known that Naive treated nematodes are attracted to salt. Also, as a chemotaxis index, it is a reasonable reaction that NaCl treated nematodes have a lower value than Naive treated nematodes. As shown in the results of the salt concentration chemotaxis index in Fig. 10B, there was no difference in values between the CB test group and the normal feed group in Naive treated nematodes, but in NaCl treated nematodes, the CB test group had a lower value than the normal feed group, indicating that the avoidance behavior towards salt concentration during starvation in the CB test group tended to be stronger than in the normal feed group. From this result, it was shown that the intake of CB maintains and/or enhances memory and learning ability.

From the results of Examples 2 to 11, it is confirmed that the composition disclosed herein has the effect of promoting adult neurogenesis. Specifically, adult neurogenesis refers to neurogenesis occurring in the adult brain, i.e., after early developmental periods (fetal and infant). In the present disclosure, from the perspective of adult neurogenesis, factors expressed in each process of neurogenesis described in non-patent literature 8 (Fig. 10 therein) were verified. Specifically, in Example 2, a significant increase in the division and proliferation of neural stem cells, which is the starting point of differentiation, was confirmed by the amount of BrdU labeling, and in Example 3, a significant increase in the number of NeuN-positive cells, a marker of mature neurons, was confirmed, indicating that the composition disclosed herein promotes adult neurogenesis. Furthermore, to elaborate, the increase in NeuroD1 and subsequent Calretinin expression-positive cells, which are expressed in the process of differentiation into neural cells, was confirmed in Examples 5 and 4, respectively. Furthermore, Example 6 confirmed that the disclosed composition exerts a promotion effect beyond what is conventionally assumed for adult neurogenesis, namely by increasing the number of quiescent neural stem cells that underlie adult neurogenesis. On the other hand, it was confirmed in Examples 7 and 8 that the composition disclosed herein promotes not only the differentiation into neurons but also the activation of microglia and astrocytes. The effects of individual bacterial strains were shown in Examples 9 to 11. It was confirmed in Example 10 that lactic acid bacteria alone promote the proliferation of human neural stem cells. Additionally, it was confirmed in Example 11 that any of amylolytic bacteria, lactic acid bacteria, and butyrate-producing bacteria alone promote the differentiation of human neural stem cells into neurons.

The results of Examples 2-12 demonstrate that the composition maintains homeostasis of brain function. Specifically, non-patent literature 9 classifies the morphological changes of microglia in detail, and non-patent literature 10 describes the function of microglia in maintaining homeostasis. That is, microglia play an important role in maintaining homeostasis of brain function by controlling axon fasciculation, programmed cell death, formation of neural processes, synapse homeostasis ("pruning"), synapse formation, and maintaining the function of neurons in the brain. Furthermore, microglia control the proliferation and differentiation of neural precursor cells (NPC) and oligodendrocyte precursor cells (OPC), promote myelin formation, and increase the activation and proliferation of astrocytes. Additionally, microglia play a role in eliminating unnecessary cells and foreign substances through phagocytosis. Astrocytes have various functions, including maintaining the central nervous system, uptake and recycling of neurotransmitters, supporting the metabolism of neurons, and maintaining the blood-brain barrier. The interaction with microglia is also described in detail in non-patent literature 11. Specifically, from the results of microscopic observation in Examples 7 and 8 of the composition of the present disclosure (based on changes in cell area), the activation of microglia and astrocytes was evidenced, indicating their contribution to the maintenance of homeostasis of brain function. Furthermore, from the results of Example 12, it was confirmed that the intake of butyrate-producing bacteria maintains and/or enhances memory and learning.

### Industrial Applicability

This disclosure can provide a composition for: increasing the number of quiescent neural stem cells; promoting the proliferation of neural stem cells; promoting the differentiation and maturation of neural stem cells into neurons; promoting the maturation of immature neurons; promoting the activation of microglia; promoting the activation of astrocytes; promoting adult neurogenesis; maintaining homeostasis of brain function; and maintaining and/or improving memory and/or learning.

## Claims

1. A composition comprising one or more bacteria selected from the group consisting of lactic acid bacteria, butyrate-producing bacteria, and amylolytic bacteria, for use in one or more of the following:
1) For increasing the number of quiescent neural stem cells;
2) For promoting the proliferation of neural stem cells;
3) For promoting the differentiation and maturation of neural stem cells into neurons;
4) For promoting the maturation of immature neurons;
5) For promoting adult neurogenesis;
6) For maintaining and/or improving memory and/or learning ability;
7) For promoting the activation of microglia;
8) For promoting the activation of astrocytes; and
9) For maintaining homeostasis of brain function.

2. The composition according to claim 1, comprising two or more bacteria selected from the group consisting of lactic acid bacteria, butyrate-producing bacteria, and amylolytic bacteria.

3. The composition according to claim 1, comprising lactic acid bacteria, butyrate-producing bacteria, and amylolytic bacteria.

4. The composition according to any one of claims 1 to 3, wherein the lactic acid bacteria are of the genus Enterococcus, the butyrate-producing bacteria are of the genus Clostridium, and the amylolytic bacteria are of the genus Bacillus.

5. The composition according to any one of claims 1 to 3, wherein the lactic acid bacteria are of the species Enterococcus faecium or Enterococcus lactis, the butyrate-producing bacteria are of the species Clostridium butyricum, and the amylolytic bacteria are of the species Bacillus subtilis.

6. The composition according to any one of claims 1 to 3, wherein the lactic acid bacteria are Enterococcus faecium strain T-110, the butyrate-producing bacteria are Clostridium butyricum strain TO-A, and the amylolytic bacteria are Bacillus subtilis strain TO-A.

7. A composition for increasing the number of quiescent neural stem cells, comprising bacteria of the species Enterococcus faecium or Enterococcus lactis, Clostridium butyricum, and Bacillus subtilis.

8. A composition for promoting the differentiation and maturation of neural stem cells into neurons, comprising bacteria of the species Enterococcus faecium or Enterococcus lactis, Clostridium butyricum, and Bacillus subtilis.

9. A composition for promoting the proliferation of neural stem cells, comprising bacteria of the species Enterococcus faecium or Enterococcus lactis, Clostridium butyricum, and Bacillus subtilis.

10. A composition for promoting the maturation of immature neurons, comprising bacteria of the species Enterococcus faecium or Enterococcus lactis, Clostridium butyricum, and Bacillus subtilis.

11. A composition for promoting the activation of microglia, comprising bacteria of the species Enterococcus faecium or Enterococcus lactis, Clostridium butyricum, and Bacillus subtilis.

12. A composition for promoting the activation of astrocytes, comprising bacteria of the species Enterococcus faecium or Enterococcus lactis, Clostridium butyricum, and Bacillus subtilis.

13. A composition for promoting adult neurogenesis, comprising bacteria of the species Enterococcus faecium or Enterococcus lactis, Clostridium butyricum, and Bacillus subtilis.

14. A composition for maintaining homeostasis of brain function, comprising bacteria of the species Enterococcus faecium or Enterococcus lactis, Clostridium butyricum, and Bacillus subtilis.

15. A composition for maintaining and/or improving memory and/or learning, comprising butyrate-producing bacteria.

16. The composition according to claim 15, wherein the butyrate-producing bacteria are of the genus Clostridium.

17. The composition according to claim 15, wherein the butyrate-producing bacteria are Clostridium butyricum TO-A.
